# FASCICULE DE BREVET EUROPEEN

(11) **EP 4 241 044 B1**
(45) Date de publication et mention de la délivrance du brevet: **25.02.2026**
(21) Numéro de dépôt: 21815551.3
(22) Date de dépôt: 21.10.2021
(51) Int. Cl.: G01C 21/20, A61F 9/02, A63B 33/00, B63C 11/12

(54) **SYSTEME D'AIDE AU GUIDAGE D'UN UTILISATEUR SE DIRIGEANT VERS OU OBSERVANT UNE ZONE D'INTERET DETERMINEE**
BENUTZERFÜHRUNGSASSISTENZSYSTEM ZUR AUSRICHTUNG AUF ODER BEOBACHTUNG EINER BESTIMMTEN INTERESSENSZONE
USER GUIDANCE ASSISTANCE SYSTEM DIRECTING TOWARDS OR OBSERVING A DETERMINED ZONE OF INTEREST

(30) Priorité: 05.11.2020 FR 2011341
(43) Date de publication de la demande: 13.09.2023
(73) Titulaire: Peripheral, 27200 Vernon (FR)
(72) Inventeur: DE LA CHOUE DE LA METTRIE, Aymar, 27200 Vernon (FR)
(74) Mandataire: RVDB
(86) Numéro de dépôt international: PCT/FR2021/051844
(87) Numéro de publication internationale: WO 2022/096797

(56) Documents cités:
- WO-A1-2015/020995
- WO-A1-2015/038527
- DE-A1- 102009 060 683
- US-A1- 2014 223 647
- US-A1- 2017 287 441
- US-B1- 10 012 506
- KISS FRANCISCO FRANCISCO KISS@VIS UNI-STUTTGART DE ET AL: "Clairbuoyance Improving Directional Perception for Swimmers", HUMAN FACTORS IN COMPUTING SYSTEMS, ACM, 2 PENN PLAZA, SUITE 701NEW YORKNY10121-0701USA, 2 May 2019 (2019-05-02), pages 1 - 12, XP058574593, ISBN: 978-1-4503-5971-9, DOI: 10.1145/3290605.3300467

## Description

### Domaine technique

La présente invention concerne le domaine des systèmes d'aide au guidage.

L'objet de la présente invention porte plus particulièrement sur un système d'aide au guidage permettant un affichage d'informations visuelles en temps réel pour aider un utilisateur à observer une zone d'intérêt déterminée ou se diriger vers une zone d'intérêt déterminée.

Par zone d'intérêt au sens de la présente invention, on entend dans toute la description qui suit toute zone qui présente un intérêt pour l'utilisateur ; il peut s'agir par exemple d'une destination finale comme un point de rassemblement/ralliement, une ligne d'arrivée, une piste d'atterrissage. Il peut également s'agir d'un trajet, d'une route prédéfinie ou encore d'une ou plusieurs étapes sur un trajet ou une route prédéfinie. Il peut aussi s'agir d'une cible ou d'un point ou une personne à observer/surveiller. On comprendra ici que, généralement, cette zone d'intérêt est définie par un ensemble de coordonnées spatiales.

La présente invention trouve de nombreuses applications avantageuses dans le domaine du sport et notamment les sports extrêmes ou les sports de glisse comme par exemple le saut en parachute, le parapente, la descente en vélo tout-terrain, le ski, le rallye automobile, etc.

La présente invention trouve également de nombreuses autres applications avantageuses dans d'autres activités comme par exemple pour le pilotage d'un véhicule (avion ou autre) ou pour les interventions de pompiers ou de militaires dans un environnement hostile.

### Art antérieur

On connaît dans l'art antérieur les interfaces déportées ; ces interfaces déportées se situent par définition en dehors du champ de vision de l'utilisateur et requiert un mouvement de tête de l'utilisateur pour être consultées.

On comprend aisément que l'utilisation de telles interfaces n'est pas envisageable pour certaines applications comme par exemple la pratique d'un sport extrême. Le sportif a en effet besoin d'être focalisé sur sa pratique pour éviter tout accident ; il en est de même du pompier ou du militaire lors d'une opération car ni l'un ni l'autre ne peut utiliser ses mains pour interagir avec l'écran.

On connaît dans l'art antérieur d'autres types d'interfaces qui se situent dans le champ de vision de l'utilisateur.

Dans l'aviation et plus récemment dans l'automobile, de telles interfaces se présentent sous la forme de dispositifs d'affichage tête haute également connus sous l'acronyme HUD (de l'anglais « Head Up Display »).

Ces dispositifs d'affichage tête haute intègrent le plus souvent des moyens d'affichage en réalité augmentée qui permettent de fournir à l'utilisateur des informations additionnelles comme par exemple des informations extrinsèques portant sur l'environnement du véhicule.

L'affichage de ces informations extrinsèques contribue bien souvent à la sécurité du véhicule et permet au conducteur ou au pilote d'appréhender l'environnement dans lequel celui-ci évolue en améliorant sa perception de l'environnement.

Ainsi, dans le domaine de l'automobile, on connaît déjà certains systèmes ADAS (de l'anglais « Advanced Driver-Assistance System » ou en français « Système d'aide à la conduite avancé ») qui sont munis d'une caméra embarquée et qui sont configurés pour afficher sur un écran, par exemple un écran tête haute ou HUD, un élément de l'environnement routier tel que par exemple un panneau de signalisation routière de l'environnement routier et/ou un pictogramme représentatif d'un véhicule circulant devant ledit véhicule ou un danger détecté.

On connaît également d'autres casques ou lunettes intégrant un écran comme par exemple ceux proposés dans les documents EP3028121B1, FR3061312A1, FR3017966A1, US2003/0184868A1, US6,394,601B1ou encore FR 2806312A1.

Ces dispositifs apportent donc des informations additionnelles soit décalées soit en superposition du champ de vision de l'utilisateur.

Le principe de superposer des informations additionnelles dans le champ de vision d'un utilisateur rend toutefois l'usage de ce type de dispositif impropre à certaines applications.

Le Demandeur soumet en effet que la pratique intensive comme la pratique d'un sport extrême ou une intervention par des forces de sécurité nécessite la fourniture d'une information simple et intuitive pour l'utilisateur. Cette information ne doit pas venir obstruer le champ de vision de l'utilisateur et doit être exploitable par l'utilisateur sans trop de charge cognitive. En effet, les informations explicites, les chiffres, les lettres demandent à l'utilisateur de se défocaliser de l'activité sur laquelle il est concentré.

Il est par ailleurs préférable que le casque soit léger et peu encombrant afin d'éviter de gêner la pratique ou de créer des troubles ou des fatigues sur le cou de l'utilisateur.

Il est également préférable que les temps de calcul soient très courts, ceci notamment pour fournir une information en temps réel et garantir une bonne autonomie énergétique.

Le Demandeur soumet respectueusement que les solutions de l'art antérieur ne sont pas satisfaisantes et n'offrent pas de système à la fois ergonomique et léger, nécessitant peu de ressources informatiques et fournissant à l'utilisateur une information visuelle fiable et intuitive sans obstruer son champ de vision ni gêner la pratique. D'autres solutions pour naviguer un utilisateur vers une zone d'intérêt sont connues des documents US 2017/287441 A1, US 10 012 506 B1 et JP 2005 069759 A.

### Résumé de l'invention

La présente invention vise à améliorer la situation décrite ci-dessus.

Un des objectifs de la présente invention est notamment de remédier aux différents inconvénients mentionnés ci-dessus en proposant une solution d'aide au guidage d'un utilisateur se dirigeant vers ou observant une zone d'intérêt.

A cet effet, l'objet de la présente invention concerne selon un premier aspect un système d'aide au guidage d'un utilisateur se dirigeant vers ou observant une zone d'intérêt déterminée selon la revendication 1.

Dans un autre mode de réalisation avantageux de la présente invention, l'écran optique de visualisation est pourvu de gravures et/ou d'inclusions formées dans l'écran, par exemple verticalement et/ou horizontalement, pour conduire la lumière émise par l'au moins une source lumineuse sur l'écran, de préférence vers le centre du champ de vision de manière à recentrer l'information pour que celle-ci soit moins dans la zone de vision périphérique.

Selon l'invention, il est prévu que l'unité de pilotage soit configurée pour piloter les sources lumineuses de manière à former un réticule virtuel dans le champ de vision de l'utilisateur.

On comprend ici que les sources lumières sont actionnées sélectivement sur le pourtour périphérique de la fenêtre de visualisation de manière à s'interposer les unes par rapport aux autres pour se croiser et former un réticule virtuel dans l'axe de champ de vision de l'utilisateur, un tel réticule virtuel indiquant avec précision la direction/orientation de la zone cible à observer ou atteindre.

On parle ici de réticule virtuel car les sources lumineuses sont ici sur le pourtour périphérique de la fenêtre de visualisation et ne viennent pas dans l'axe central du champ de vision de l'utilisateur. La présence des sources lumineuses actionnées sélectivement en périphérie permet la formation d'un réticule non réel qui est perçu par le cerveau de l'utilisateur par extrapolation des sources lumineuses et qui est formé fictivement par le cerveau de l'utilisateur dans le champ de vision sans gêner la concentration et la vision de celui-ci.

Préférentiellement, l'au moins un capteur est un capteur de position et/ou un capteur d'azimut et/ou un capteur d'inclinaison et/ou un accéléromètre.

Un tel capteur permet ainsi de récupérer une information relative à la position relative de la fenêtre de visualisation par rapport à la zone d'intérêt, l'azimut et/ou l'inclinaison de la fenêtre de visualisation.

Avantageusement, le dispositif d'affichage comprend des moyens de communication sans fil configurés pour communiquer avec une entité externe afin de récupérer au moins une information externe relative à l'environnement externe de l'utilisateur.

Avantageusement, le dispositif d'affichage comprend des moyens d'acquisition configurés pour acquérir au moins une information d'état du système.

Préférentiellement, l'unité de pilotage est configurée pour contrôler chacune des sources lumineuses en intensité et/ou en couleur.

Préférentiellement, la pluralité de sources lumineuses comprend un ruban LED ou OLED.

Préférentiellement, la pluralité de sources lumineuses comprend une fibre optique.

Corrélativement, l'objet de la présente invention concerne selon un deuxième aspect un support facial destiné à être porté directement ou indirectement sur la tête de l'utilisateur et comprenant un système d'aide au guidage tel que décrit ci-dessus dans lequel la fenêtre de visualisation est assemblée solidairement audit support facial.

Avantageusement, le support facial est constitué d'une paire de lunette, d'un masque ou d'un casque. On comprend ici que le support facial peut également se présenter sous la forme d'une visière par exemple.

Avantageusement, le support facial est pourvu d'au moins une sangle de maintien en position dudit support sur la tête dudit utilisateur.

Ainsi, l'objet de la présente invention, par ses différents aspects fonctionnels et structurels décrits ci-dessus, permet de fournir en temps réel une information de guidage fiable à destination de l'utilisateur pour le guider ou orienter son regard vers une zone d'intérêt prédéterminée.

### Description des figures

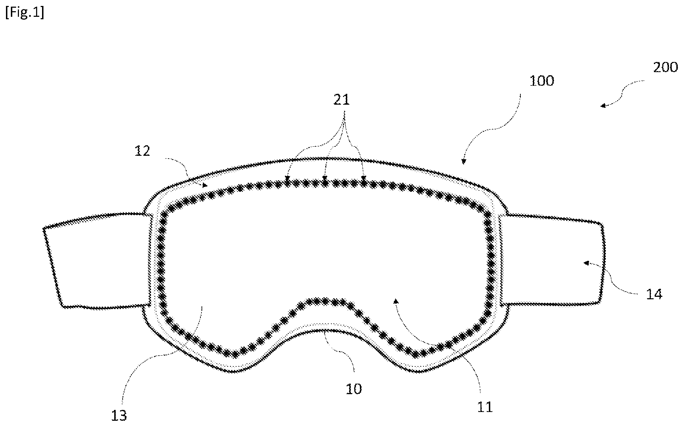

D'autres caractéristiques et avantages de la présente invention ressortiront de la description ci-dessous, en référence aux figures 1 à 9 annexées qui en illustrent différents exemples de réalisation dépourvus de tout caractère limitatif et sur lesquelles :
[Fig.1]
   La figure 1 représente une première vue schématique d'un système d'aide au guidage selon un exemple de réalisation de la présente invention ;
[Fig.2]

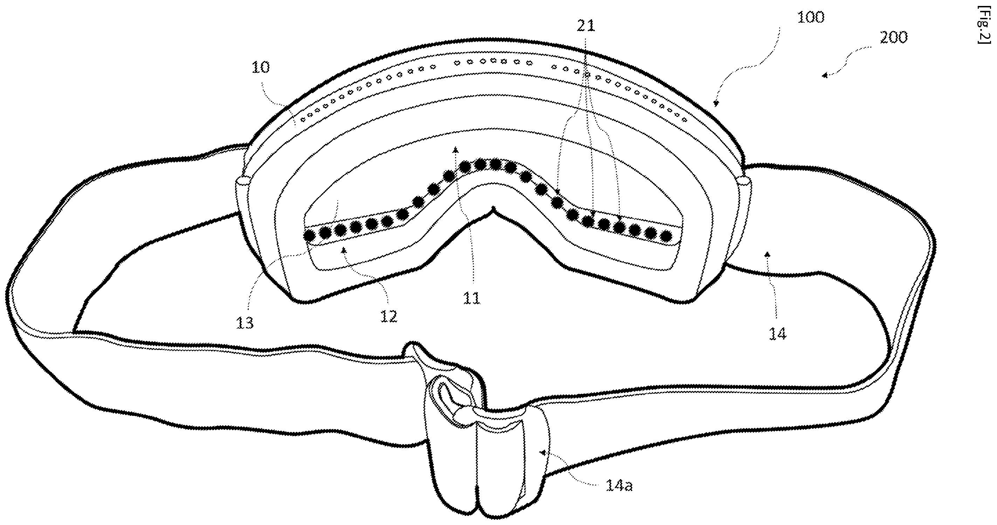

   La figure 2 représente une deuxième vue schématique du système d'aide au guidage conforme à la figure 1 ;
[Fig.3]

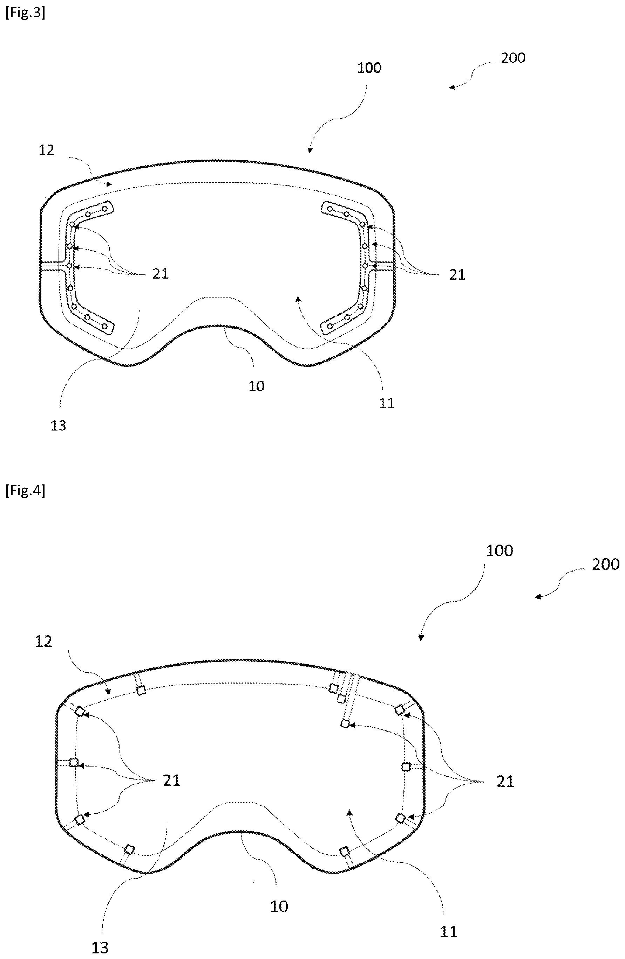

   La figure 3 représente une vue schématique d'un système d'aide au guidage selon un autre exemple de réalisation de la présente invention ;
[Fig.4]
   La figure 4 représente une vue schématique d'un système d'aide au guidage selon un autre exemple de réalisation de la présente invention ;
[Fig.5]

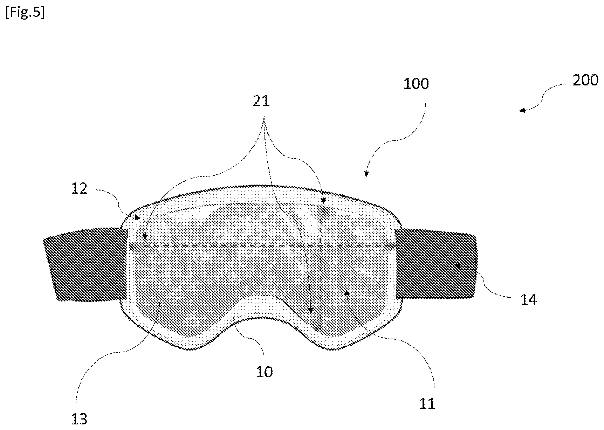

   La figure 5 représente une vue schématique d'un système d'aide au guidage formant un réticule dans le champ de vision de l'utilisateur ;
[Fig.6]

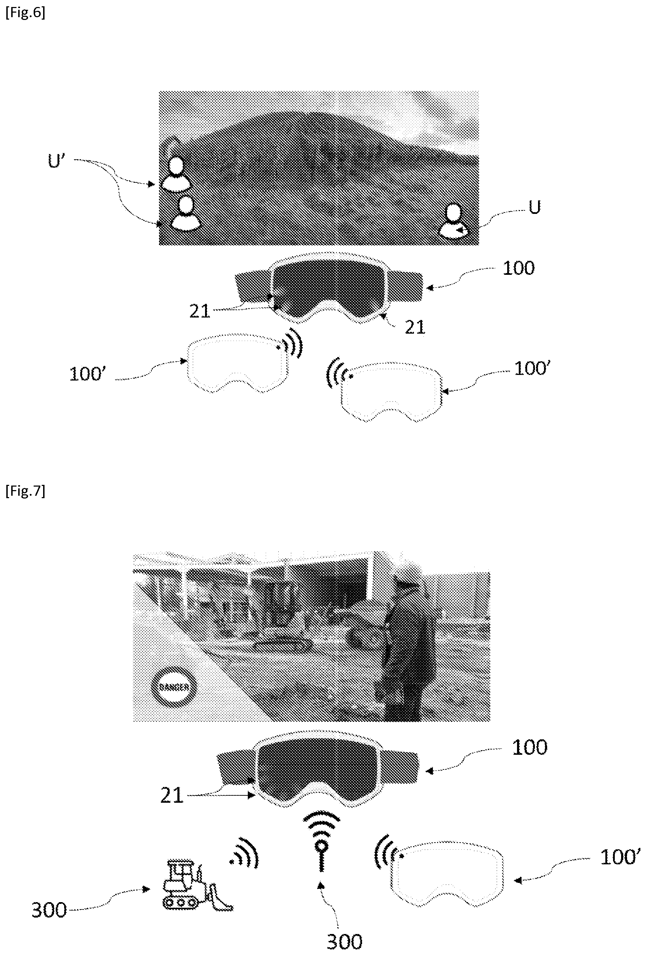

   La figure 6 représente une vue schématique représentant une utilisation d'un système d'aide au guidage selon l'invention pour la pratique d'une activité avec plusieurs utilisateurs ;
[Fig.7]
   La figure 7 représente une vue schématique représentant une utilisation d'un système d'aide au guidage selon l'invention pour un chantier comprenant des dangers ;
[Fig.8]

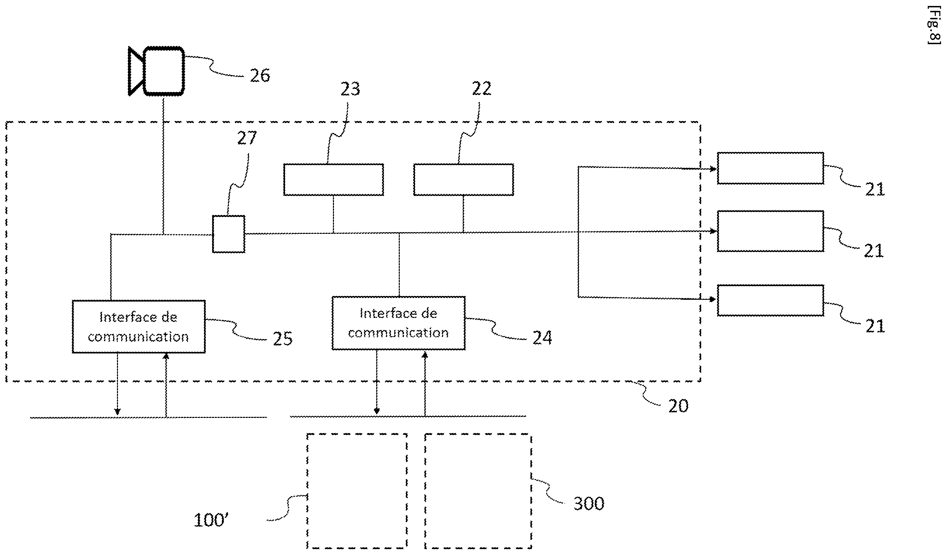

   La figure 8 illustre schématiquement le dispositif d'affichage d'information de guidage intégré dans le système d'aide au guidage selon un exemple de réalisation particulier de la présente invention ; et
[Fig.9]

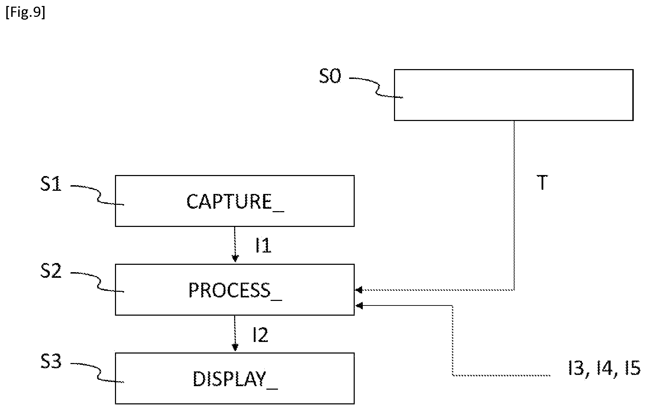

   La figure 9 illustre un organigramme des différentes étapes d'un procédé mis en œuvre par un dispositif conforme à la figure 8.

### Description des exemples de réalisation

Un système d'aide au guidage 100 ainsi que le procédé qui lui est associé vont maintenant être décrits dans ce qui va suivre en référence conjointement aux figures 1 à 9.

Des mêmes éléments sont identifiés avec des mêmes signes de référence tout au long de la description qui va suivre.

Comme expliqué en préambule, les comportements se digitalisent et tirent de plus en plus profit de la valorisation des données en particulier géolocalisées.

Pourtant les technologies connues jusqu' à présent et permettant d'accéder à la réalité augmentée excluent les utilisateurs sous contraintes dont la sécurité dépend essentiellement d'informations simples et intuitives, comme par exemple les personnes pratiquant un sport extrême ou des pompiers ou des forces de sécurité.

Le Demandeur observe en effet qu'une personne pratiquant un sport extrême comme par exemple le saut en parachute ou la descente en vélo tout-terrain doit pouvoir disposer d'une information fiable et facile à traiter sans être gêné dans sa pratique, c'est-à-dire manipuler un objet dédié ou se focaliser sur autre chose que sa trajectoire. Cette personne doit en effet se focaliser sur sa pratique et doit pouvoir obtenir des informations simples qui répondent principalement aux questions suivantes : « où dois-je aller ? », « où est le danger ? » et dans certaines situations « où sont les autres ? ».

Le Demandeur soumet que les dispositifs d'affichage tête haute (ou HUD) et les casques de réalité augmentée ne sont pas adaptés à ces problématiques et souffrent notamment de leur haute technicité ; ainsi, les solutions actuelles présentent les inconvénients suivants :
- elles nécessitent des réglages précis (distance inter-pupillaire, dioptrie binoculaire, etc.),
- elles sont lourdes et encombrantes,
- elles requièrent de l'énergie,
- elles sont coûteuses,
- elles sont susceptibles de se décaler de l'axe de vision en cas de chocs et de proposer un guidage erroné, elles sont sensibles à la buée, la fumée, la présence de poussières.

Le Demandeur soumet de plus que les solutions de l'art antérieur ne permettent pas de fournir à l'utilisateur une information simple et exploitable directement pour répondre aux questions ci-dessus.

Le système 100 proposé ici dans le cadre de la présente invention atteint cet objectif.

Pour atteindre celui-ci, le concept sous-jacent à la présente invention est d'orienter le regard et la pratique de l'utilisateur en exploitant les réflexes humains de l'attention et de l'hypervigilance par l'incrustation de points lumineux en périphérie du champ de vision de l'utilisateur.

L'exploitation du champ de vision périphérique est caractéristique de la présente invention.

Une telle exploitation du champ de vision périphérique permet une conscience visuelle périphérique de l'utilisateur laquelle est considérée comme la capacité d'être conscient sans être distrait par une quantité importante d'information par rapport au cadre particulier et à la tâche à accomplir.

Sur le plan physiologique, on sait que la zone de vision périphérique représente 80% de la surface de la rétine pour 20% des cellules optiques. Le champ de vision périphérique permet donc la mise en place d'un processus de vigilance sur une grand portion d'espace, avec une vision floue et déficiente de la lumière, sans distraire de la tâche principale.

On sait de plus que les situations stressantes (comme c'est le cas d'usage dans le cas de la présente invention) éveillent le système nerveux qui, entre autres, provoque une dilatation des pupilles. Une telle dilatation de la pupille permet donc à plus de lumière d'entrer et d'atteindre une plus grande zone de la rétine, ce qui peut fournir un plus large éventail d'informations sur l'environnement.

C'est donc ici ce champ de vision périphérique qu'on cherche à utiliser pour fournir à l'utilisateur une information simple et intuitive sans le distraire dans sa concentration.

La présente invention prévoit dans l'exemple des figures 1 et 2, qui en illustrent un exemple de réalisation particulier et non limitatif de la présente invention, la mise en œuvre d'un système d'aide au guidage 100 d'un utilisateur U se dirigeant vers une zone d'intérêt, notée ici T (pour « *target* »).

Cette zone d'intérêt T peut correspondre par exemple à un point de rendez-vous ou de ralliement, une ligne d'arrivée, une étape sur un trajet, un point particulier à observer, une zone de danger à éviter etc.

Ici, cette zone d'intérêt T est donc définie par des coordonnées spatiales qui sont connues.

Le système 100 comprend un dispositif 10, appelé ici dispositif d'affichage, qui comporte une carte électronique ayant au moins une mémoire 27.

Les coordonnées de la zone d'intérêt T sont enregistrées au préalable lors d'une étape S0 dans cette mémoire 27.

Dans cet exemple, l'au moins une mémoire 27 correspond par exemple à une mémoire volatile et/ou non volatile et/ou comprend un dispositif de stockage mémoire qui peut comprendre de la mémoire volatile et/ou non volatile, telle que EEPROM, ROM, PROM, RAM, DRAM, SRAM, flash, disque magnétique ou optique.

Comme énoncé précédemment, un des objets de la présente invention est de fournir une information facilement exploitable permettant à l'utilisateur U d'être guidé en temps réel et de façon intuitive vers cette zone d'intérêt T sans être gêné dans la pratique de son activité. Dans cet exemple et comme illustré en figure 1 et 2, le système 100 intégré directement ou indirectement dans un support facial 200 se présentant sous la forme d'un masque comme par exemple un masque de ski.

Dans l'exemple la figure 2, un tel masque 200 est muni d'une sangle 14 destinée à maintenir le masque 200 en position autour de la tête de l'utilisateur U.

Dans cet exemple, la sangle 14 est en outre pourvue de moyens de réglage 14a permettant de régler le diamètre de la sangle 14 pour s'adapter à la taille de la tête de l'utilisateur U.

Dans cet exemple, on utilise un masque 200 classique dont la structure générale épouse l'anatomie du visage de l'utilisateur U.

Dans cet exemple, le système 100 intégré au masque 200comprend donc une fenêtre de visualisation 11 délimitée par le pourtour périphérique 12 lequel correspond ici au cadre du masque 200.

Le pourtour périphérique 12 de cette fenêtre 11 définit ici le champ de vision périphérique de l'utilisateur U.

Dans cet exemple, il est prévu un écran optique de visualisation 13, ici un écran protecteur, couvrant le champ de vision et permettant de protéger les yeux de l'utilisateur U.

Un tel écran 13 peut en outre comprendre un traitement antibuée, un filtre anti-ultraviolets et/ou un traitement antireflet pour améliorer le confort de l'utilisateur.

Dans cet exemple et comme illustré en figures 1 et 2, le pourtour périphérique 12 est équipé d'une pluralité de sources lumineuses 21. Ces sources lumineuses 21 se situent donc en périphérie du champ de vision de l'utilisateur U et ne sont pas susceptibles de gêner celui-ci dans sa perception de l'espace. Elles restent donc visibles sans obstruer la vision (c'est-à-dire sans venir dans l'axe central du champ de vision de l'utilisateur).

Dans l'exemple illustré en figures 1 et 2, les sources lumineuses 21 s'étendent à intervalles réguliers le long de ce pourtour périphérique 12.

Ici, les sources lumineuses 21 sont formées par un bandeau de LED (de l'anglais « Light-Emitting Diode » ou en français « Diode électroluminescente ») ou d'OLED (de l'anglais « Organic Light-Emitting Diode » ou en français « Diode électroluminescente organique »).

D'autres types de sources lumineuses 21 peuvent également être envisagés ici par l'homme du métier comme par exemple de la fibre optique.

Un des concepts sous-jacents à la présente invention est d'utiliser ces sources 21 pour indiquer en dehors de l'axe principale du champ de vision de l'utilisateur U (mais en périphérie de celui-ci) la direction préférée à suivre pour atteindre la zone d'intérêt T.

Ceci est rendu possible par une intelligence embarquée dans le système 100.

Cette intelligence réside plus particulièrement dans le dispositif 10 lequel comprend, sans y être limité, un équipement électronique embarqué tel qu'un calculateur électronique (une UCE pour « Unité de Commande Electronique », un téléphone intelligent, une tablette ou encore un ordinateur portable). Les éléments du dispositif 10, individuellement ou en combinaison, peuvent être intégrés dans un unique circuit intégré, dans plusieurs circuits intégrés, et/ou dans des composants discrets. Le dispositif 10 peut être réalisé sous la forme de circuits électroniques ou de modules logiciels (ou informatiques) ou encore d'une combinaison de circuits électroniques et de modules logiciels.

Dans cet exemple, le dispositif 10 comprend un (ou plusieurs) processeur(s) configurés pour exécuter des instructions pour la réalisation des étapes du procédé et/ou pour l'exécution des instructions du ou des logiciels embarqués dans le dispositif 10. Le processeur peut inclure de la mémoire intégrée, une interface d'entrée/sortie, et différents circuits connus de l'homme du métier.

Le code informatique du ou des logiciels embarqués comprenant les instructions à charger et exécuter par le processeur est par exemple stocké sur la mémoire 27.

Dans l'exemple décrit ici, le dispositif 20 comprend en outre une unité de pilotage 22 capable de piloter les sources 21 indépendamment les unes des autres.

Dans cet exemple, on prévoit également l'intégration d'au moins un capteur 23 tel qu'un capteur d'azimut, un capteur d'inclinaison et/ou un capteur de position. Un tel capteur 23 est capable de capturer lors d'une étape S1 une information spatiale I1 relative à une position et/ou une orientation de la fenêtre de visualisation (11) par rapport à ladite zone d'intérêt T.

Le capteur 23 mesure donc une valeur représentative de la position relative du masque (c'est-à-dire l'inclinaison/orientation/position du regard de l'utilisateur) par rapport à la zone d'intérêt T à atteindre.

Cette information spatiale I1 est ensuite transmise au circuit de traitement 24 (ici par exemple un calculateur) qui lors d'une étape S2 va traiter cette information spatiale I1 en fonction de la zone d'intérêt T (ici les coordonnées spatiales de la zone d'intérêt T enregistrées dans la mémoire 27) afin de déterminer une information de guidage I2.

Cette information I2 vise à déterminer un vecteur de guidage pour orienter le regard de l'utilisateur U de manière à ce que celui-ci observe ou se dirige vers la zone d'intérêt T.

Cette information de guidage I2 est alors envoyée à l'unité de pilotage 22 pour que celle-ci pilote sélectivement chacune des sources lumineuses 21 en fonction de cette information de guidage I2 de manière à, lors de l'étape S3, afficher en périphérie du champ de vision de l'utilisateur une direction à suivre pour que celui-ci se dirige vers ladite zone d'intérêt T.

On comprend donc ici que les sources lumineuses 21 vont s'éclairer en fonction de cette information de guidage I2 déterminée par le circuit de traitement 24.

Ce pilotage sélectif des sources lumineuses 21 en dehors de l'axe central du champ de vision de l'utilisateur mais en périphérie de celui-ci permet de guider l'utilisateur U vers la zone d'intérêt T sans gêner celui-ci.

Ainsi, comme illustré en figure 5, les sources lumineuses sont actionnées en périphérie du champ de vision de l'utilisateur de manière à former un réticule virtuel dans le champ de vision de celui-ci. Ce dernier n'a plus qu'à suivre la direction fournie par le réticule virtuel en fixant son regard vers le croisement fictif de deux faisceaux lumineux formés par les deux paires de sources lumineuses activées.

On comprendra ici que cette information évolue en temps réel avec le mouvement de la tête et donc de l'axe central de vision de l'utilisateur.

L'affichage d'une information visuelle simple à la périphérie du champ de vision de l'utilisateur selon la direction du regard et la position de l'utilisateur est caractéristique de la présente invention.

L'affichage de cette information située dans la zone de vision périphérique permet à l'utilisateur de s'orienter intuitivement dans l'espace.

Plusieurs autres exemples de réalisation alternatif sont décrits et illustrés en figures 3 et 4.

En figure 3, on retrouve l'ensemble des caractéristiques des figures 1 et 2 décrites ci-dessus. Le positionnement et l'agencement des sources lumineuses 21 sont toutefois très légèrement différents de l'exemple des figures 1 et 2.

Ici, les sources lumineuses 21 sont intégrées directement dans l'écran 13.

Ces sources 21 sont insérées dans une ou plusieurs couches et à différents écartements de l'axe central de vision entre ou sur les différentes feuilles composant l'écran 13.

Dans cet exemple, les sources 21 ne s'étendent que sur les bords latéraux de l'écran 13 de manière à limiter l'encombrement du champ de vision. On pourra considérer ici que les sources 21 restent donc en périphérie du champ de vision au sens de la présente invention et ne viennent pas dans l'axe central du champ de vision.

En figure 4, un autre exemple de réalisation est encore illustré.

Dans celui-ci, il est prévu une inclusion d'une ou plusieurs sources 21 à une position spécifique, ici par exemple en haut à gauche du champ de vision de l'utilisateur U. Une telle inclusion peut être réalisée par exemple par un ou plusieurs guides lumineux de type fibre optique et permet de réfléchir la lumière vers l'axe de vision central du regard de l'utilisateur U. Une telle source lumineuse 21 incluse dans l'écran 13 peut être porteuse d'une information à caractère important comme par exemple une information relative à un danger imminent.

Dans un mode de réalisation avantageux non illustré ici, il est possible de former dans l'écran optique de visualisation 13 des gravures et/ou des inclusions rectilignes formées par exemple verticalement et/ou horizontalement dans l'écran 13 afin de pour conduire la lumière émise par l'au moins une source lumineuse 21 sur l'écran 13.

Grâce à ces gravures/inclusions ménagées dans l'écran 13, il est ainsi possible de former dans le champ de vision un réticule réel dans l'axe du champ de vision de l'utilisateur lequel permet de guider avec précision l'utilisateur vers la zone T.

Afin d'enrichir l'information visuelle communiquée à l'utilisateur U, il est prévu de fournir d'autres informations et de tenir compte de l'environnement externe (présence d'un danger par exemple) et des éventuels autres utilisateurs U' qui se situent à proximité de l'utilisateur U.

On prévoit donc d'équiper le dispositif 20 de moyens de communication sans fil 24 pour communiquer avec d'autres entités externes, par exemple un autre système 100' d'un autre utilisateur U' (figure 6), un serveur distant, le « cloud », une entité 300 comme par exemple un camion (figure 7) susceptible de représenter un danger ou encore une borne signalant une zone de danger à ne pas franchir sur un chantier.

Ces moyens de communication 24 sont donc capables de communiquer avec un ou plusieurs dispositifs externes 100' et/ou 300 et comprennent une ou plusieurs des interfaces radiofréquence RF, par exemple de type Bluetooth^{®} ou Wi-Fi^{®}, LTE (de l'anglais « Long-Term Evolution » ou en français « Evolution à long terme »), LTE-Advanced (ou en français LTE-avancé). Ainsi, des données peuvent par exemple être chargées vers le dispositif 10 via l'interface de communication 24 en utilisant un réseau Wi-Fi^{®} tel que selon IEEE 802.11, un réseau ITS G5 basé sur IEEE 802.11p ou un réseau mobile tel qu'un réseau 4G (ou LTE Advanced selon 3GPP release 10 - version 10) ou 5G, notamment un réseau LTE-V2X.

Les informations I3 recueillies par ces moyens de communication sans fil 24 sont transmises au circuit de traitement 24 et sont analysées pour être prises en considération dans la détermination de l'information de guidage I2. Par exemple, la présence d'un danger ou d'un obstacle sur le trajet de l'utilisateur U peut ainsi être prise en considération pour recalculer un autre trajet afin d'atteindre la zone d'intérêt T.

Pour sécuriser le système, on peut également prévoir l'intégration d'une caméra additionnelle 26 capable de capter des images représentatives I5 de l'environnement. Ces images I5 peuvent ensuite être traitées par des algorithmes de traitements d'images pour détecter un éventuel danger ou un obstacle.

On peut aussi prévoir l'intégration d'un ou plusieurs LIDAR(s) (de l'anglais « Light Detection And Ranging », ou « Détection et estimation de la distance par la lumière » en français) arrangés sur le casque ; un capteur LIDAR correspond ici à un système optoélectronique composé d'un dispositif émetteur laser, d'un dispositif récepteur comprenant un collecteur de lumière (pour collecter la partie du rayonnement lumineux émis par l'émetteur et réfléchi par tout objet situé sur le trajet des rayons lumineux émis par l'émetteur) et d'un photodétecteur qui transforme la lumière collectée en signal électrique ; un capteur LIDAR permet ainsi de détecter la présence d'objets situés dans le faisceau lumineux émis et de mesurer la distance entre le capteur et chaque objet détecté, ceci dans le but de récupérer des points représentatifs d'un obstacle ou d'un danger sur le trajet de l'utilisateur.

Les informations provenant de cette caméra 26 ou du/des LIDAR sont envoyées directement au circuit de traitement 24 pour analyse et prise en considération dans la détermination de l'information de guidage I2.

Les informations I3 et/ou I5 provenant de ces moyens de communication sans fil 24 et/ou de la caméra 26 et/ou du/des LIDAR peuvent également être directement affichées sur le pourtour périphérique 12 du champ de vision de l'utilisateur par l'actionnement d'une des sources lumineuses 21.

Ainsi, par exemple, quand un danger extérieur est détecté (par exemple via les moyens 24 ou la caméra 26), il est possible d'actionner une source spécifique comme par exemple celle qui se situe par inclusion dans l'écran 13 (figure 4).

Pour sécuriser le système, on peut également prévoir l'intégration de microphone et/ou d'écouteurs capables de récolter ou de traiter des sons. Ces sons peuvent ensuite être traitées par des algorithmes de traitement configurés pour réduire les bruits parasites, ou renforcer la perception spatiale d'un point géoréférencé.

Dans l'exemple décrit ici, il est également possible de commander les sources lumineuses 21 en intensité et/ou en couleur. Il est possible par exemple d'émettre dans le champ de vision une lumière rouge qui clignote avec une intensité forte pour signaler un danger imminent.

Bien évidemment, il s'agit d'un exemple parmi d'autres possibles.

L'homme du métier comprendra ici qu'il est possible de prévoir en fonction du cas d'usage une pluralité de scénarii et de programmer le pilotage des sources lumineuses 21 en fonction des spécificités de chaque pratique et de chaque besoin.

De la même façon, d'autres informations peuvent avoir leur intérêt et être communiquées via les sources lumineuses 21 à l'utilisateur U. Ainsi, il est prévu dans l'exemple décrit ici la présence de moyens d'acquisition 25 qui sont capables de récupérer des informations d'état I4 du système 100 comme par exemple l'état de la batterie d'alimentation ou le niveau d'usure de certains composants pour prévenir d'une opération de maintenance.

Selon le codage de l'information et l'implémentation du modèle décisionnel d'affichage, le dispositif 100 est capable d'afficher plusieurs informations différentes selon différents modes sélectionnables.

On peut par exemple prévoir la mise en œuvre d'une télécommande se présentant par exemple sous la forme d'une montre connectée au poignet de l'utilisateur U (non représentée ici) laquelle est capable par un actionnement sur l'écran tactile de sélectionner le type d'affichage pour basculer par exemple le système d'un mode « aide au guidage/géolocalisation » permettant de suivre différents points de rendez-vous sur un trajet prédéterminé à un mode « équipement » permettant de connaître l'état d'un équipement ou encore un mode « équipe » permettant de connaître la position relative des autres utilisateurs U'.

D'autres modes d'affichage peuvent également être envisagés pour fournir à l'utilisateur une information fiable et facilement exploitable.

Ainsi, par exemple, il est possible de prévoir pour une orientation spécialement éloignée du cône de vision de l'utilisateur, l'actionnement d'une seule source lumineuse pour indiquer l'orientation vers laquelle l'utilisateur doit faire un mouvement pour trouver la zone d'intérêt. Quand la zone T entre dans le cône de vision de l'utilisateur, il est possible de prévoir l'actionnement de quatre sources lumineuses en croisillon (figure 5) pour indiquer la direction du point relativement à celle de l'axe du regard avec un réticule virtuel.

De la même façon, comme énoncé précédemment, l'intensité et/ou la couleur de tout ou partie des sources lumineuses peuvent permettre de signaler d'autres informations comme par exemple le passage de la zone d'intérêt. La variation des paramètres relatifs à l'intensité, aux couleurs et aux nombres de sources lumineuses 21 permet de superposer plusieurs informations en fonction du cas d'usage.

La présente invention permet ainsi de pallier aux différents inconvénients de l'état de la technique en permettant la fourniture d'informations implicites dans la zone de vision périphérique de l'utilisateur.

L'affichage de ces informations qui n'intervient pas dans le champ de vision principal de l'utilisateur mais en périphérie de celui-ci sont exploitables directement par l'utilisateur et ne requiert aucune réflexion.

Grâce aux moyens de communication embarqués dans le système, ces informations peuvent également être transmises à d'autres utilisateurs.

La présente invention est particulièrement destinée de manière non limitative aux sportifs extrêmes, aux pompiers, aux opérateurs industriels ou encore aux agents des forces de défense et sécurité lors de leurs interventions. Des applications de tourisme et/ou de jeux digitaux ainsi que des applications permettant de l'apprentissage ou du renforcement des capacités par interaction avec la zone de vision périphériques peuvent être aussi envisagées.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

Il devra également être observé que les signes de références mis entre parenthèses dans les revendications qui suivent ne présentent en aucun cas un caractère limitatif ; ces signes ont pour seul but d'améliorer l'intelligibilité et la compréhension des revendications qui suivent ainsi que la portée de la protection recherchée.

## Revendications

1. Système d'aide au guidage (100) d'un utilisateur (U) se dirigeant vers ou observant une zone d'intérêt (T) déterminée, ledit système (100) comportant :
- une fenêtre de visualisation (11) présentant un pourtour périphérique (12) définissant un champ de vision périphérique dudit utilisateur (U) et étant équipée d'une visière (13) définissant un champ de vision de l'utilisateur (U), et
- un dispositif d'affichage (20) d'information de guidage (I2) comprenant :
- une pluralité de sources lumineuses (21) s'étendant au moins partiellement le long dudit pourtour périphérique (12) de la fenêtre de visualisation (11), lesdites sources lumineuses (21) étant aptes à être pilotées indépendamment les unes des autres par une unité de pilotage (22),
- au moins un capteur (23) configuré pour capturer une information spatiale (I1) relative à une position et/ou une orientation de la fenêtre de visualisation (11)) par rapport à ladite zone d'intérêt (T) ;
- un circuit de traitement (24) configuré pour traiter ladite information spatiale (I1) en fonction de ladite zone d'intérêt (T) afin de déterminer une information de guidage (I2) dudit utilisateur (U) vers ladite zone d'intérêt (T) ;
ledit système (100) étant **caractérisé en ce que** ladite unité de pilotage (22) est configurée pour piloter sélectivement lesdites sources lumineuses (21) sur le pourtour périphérique (12) de la fenêtre de visualisation (11) en fonction de ladite information de guidage (I2) de manière à former un réticule virtuel indiquant dans le champ de vision de l'utilisateur (U) une direction à suivre pour se diriger vers ou observer ladite zone d'intérêt (T).

2. Système (100) selon la revendication 1, dans lequel ladite visière (13) est pourvue de gravures et/ou d'inclusions rectilignes formées dans la visière (13) pour conduire la lumière émise par l'au moins une source lumineuse (21) sur ladite visière écran (13).

3. Système (100) selon l'une quelconque des revendications précédentes, dans lequel ledit au moins un capteur (23) est un capteur de position et/ou un capteur d'azimut et/ou un capteur d'inclinaison et/ou un accéléromètre.

4. Système (100) selon l'une quelconque des revendications précédentes, dans lequel ledit dispositif d'affichage (20) comprend des moyens de communication sans fil (24) configurés pour communiquer avec une entité externe (100', 300) afin de récupérer au moins une information externe (I3) relative à l'environnement externe dudit utilisateur (U).

5. Système (100) selon l'une quelconque des revendications précédentes, dans lequel ledit dispositif d'affichage (20) comprend des moyens d'acquisition (25) configurés pour acquérir au moins une information d'état (I4) du système (100).

6. Système (100) selon l'une quelconque des revendications précédentes, dans lequel l'unité de pilotage (22) est configurée pour contrôler chacune des sources lumineuses (21) en intensité et/ou en couleur.

7. Système (100) selon l'une quelconque des revendications précédentes, dans lequel la pluralité de sources lumineuses (21) comprend un ruban LED ou OLED.

8. Système (100) selon l'une quelconque des revendications précédentes, la pluralité de sources lumineuses (21) comprend une fibre optique.

9. Support facial (200) destiné à être porté directement ou indirectement sur la tête dudit utilisateur (U) et comprenant un système (100) selon l'une quelconque des revendications précédentes, dans lequel ladite fenêtre de visualisation (11) est assemblée solidairement audit support facial (10).

10. Support (200) selon la revendication 9, lequel se présente sous la forme d'une paire de lunette, d'un masque ou d'un casque.

11. Support (200) selon la revendication 9 ou 10, lequel est pourvu d'au moins une sangle de maintien (14) en position dudit support (10) sur la tête dudit utilisateur (U).

## Patentansprüche

1. Führungshilfesystem (100) eines Benutzers (U), der sich in Richtung eines bestimmten Bereichs von Interesse (T) bewegt oder diesen beobachtet, wobei das System (100) Folgendes umfasst:
- ein Sichtfenster (11), das einen umlaufenden Umfang (12) besitzt, der ein peripheres Sichtfeld des Benutzers (U) definiert, und mit einem Visier (13) ausgestattet ist, das ein Sichtfeld des Benutzers (U) definiert, und
- eine Anzeigevorrichtung (20) für Führungsinformationen (12), umfassend:
- eine Vielzahl von Lichtquellen (21), die sich mindestens teilweise entlang des umlaufenden Umfangs (12) des Sichtfensters (11) erstrecken, wobei die Lichtquellen (21) unabhängig voneinander von einer Steuereinheit (22) gesteuert werden können,
- mindestens einen Sensor (23), der so eingerichtet ist, dass er eine räumliche Information (I1) in Bezug auf eine Position und/oder eine Ausrichtung des Sichtfensters (11)) in Bezug auf den Bereich von Interesse (T) erhebt;
- eine Verarbeitungsschaltung (24), die so eingerichtet ist, dass sie die räumliche Information (I1) in Abhängigkeit von dem Bereich von Interesse (T) verarbeitet, um eine Führungsinformation (I2) des Benutzers (U) zu dem Bereich von Interesse (T) zu bestimmen;
wobei das System (100) **dadurch gekennzeichnet ist, dass** die Steuereinheit (22) so eingerichtet ist, dass sie die Lichtquellen (21) an dem umlaufenden Umfang (12) des Sichtfensters (11) in Abhängigkeit von den Führungsinformationen (I2) selektiv steuert, um ein virtuelles Fadenkreuz zu bilden, das in dem Sichtfeld des Benutzers (U) eine Richtung anzeigt, der zu folgen ist, um sich in Richtung des Bereichs von Interesse (T) zu begeben oder diesen zu beobachten.

2. System (100) nach Anspruch 1, wobei das Visier (13) mit Gravuren und/oder geradlinigen Einschlüssen versehen ist, die in dem Visier (13) gebildet sind, um das von der mindestens einen Lichtquelle (21) ausgestrahlte Licht auf das Sichtschutzvisier (13) zu leiten.

3. System (100) nach einem der vorhergehenden Ansprüche, wobei der mindestens eine Sensor (23) ein Positionssensor und/oder ein Azimutsensor und/oder ein Neigungssensor und/oder ein Beschleunigungsmesser ist.

4. System (100) nach einem der vorhergehenden Ansprüche, wobei die Anzeigevorrichtung (20) drahtlose Kommunikationsmittel (24) umfasst, die so eingerichtet sind, dass sie mit einer externen Einheit (100', 300) kommunizieren, um mindestens eine externe Information (I3) in Bezug auf die externe Umgebung des Benutzers (U) abzurufen.

5. System (100) nach einem der vorhergehenden Ansprüche, wobei die Anzeigevorrichtung (20) Erfassungsmittel (25) umfasst, die so eingerichtet sind, dass sie mindestens eine Zustandsinformation (I4) von dem System (100) erfasst.

6. System (100) nach einem der vorhergehenden Ansprüche, wobei die Steuereinheit (22) so eingerichtet ist, dass sie jede der Lichtquellen (21) in Intensität und/oder Farbe steuert.

7. System (100) nach einem der vorhergehenden Ansprüche, wobei die Vielzahl von Lichtquellen (21) ein LED- oder OLED-Band umfasst.

8. System (100) nach einem der vorhergehenden Ansprüche, wobei die Vielzahl von Lichtquellen (21) eine optische Faser umfasst.

9. Gesichtsträger (200), der dazu bestimmt ist, direkt oder indirekt auf dem Kopf des Benutzers (U) getragen zu werden, und der ein System (100) nach einem der vorhergehenden Ansprüche umfasst, wobei das Sichtfenster (11) fest mit dem Gesichtsträger (10) verbunden ist.

10. Träger (200) nach Anspruch 9, der in Form einer Brille, einer Maske oder eines Helms vorliegt.

11. Träger (200) nach Anspruch 9 oder 10, der mit mindestens einem Haltegurt (14) in der Position des Trägers (10) an dem Kopf des Benutzers (U) versehen ist.

## Claims

1. Guidance assistance system (100) for a user (U) directing towards or observing a determined zone of interest (T), said system (100) comprising:
- a visualisation window (11) having a peripheral rim (12) defining a peripheral field of vision of said user (U) and being equipped with an optical visualisation screen (13) defining a field of vision of the user (U), and
- a display device (20) for guidance information (12) comprising:
- a plurality of light sources (21) extending at least partially along said peripheral rim (12) of the visualisation window (11), said light sources (21) being capable of being controlled independently of one another by a control unit (22),
- at least one sensor (23) configured to capture spatial information (I1) relating to a position and/or an orientation of the visualisation window (11) in relation to said zone of interest (T);
- a processing circuit (24) configured to process said spatial information (I1) according to said zone of interest (T) in order to determine guidance information (I2) of said user (U) towards said zone of interest (T);
said system (100) being **characterised in that** said control unit (22) is configured to selectively control said light sources (21) on the peripheral rim (12) of the visualisation window (11) on the basis of said guidance information (I2) so as to form a virtual reticle in the field of vision of the user (U) indicating a direction to be followed to direct towards or observe said zone of interest (T).

2. System (100) according to claim 1, wherein said optical visualisation screen (13) is provided with straight etchings and/or inclusions formed in the screen (13) to conduct the light emitted by the at least one light source (21) on the screen (13).

3. System (100) according to any one of the preceding claims, wherein said at least one sensor (23) is a position sensor and/or an azimuth sensor and/or an inclination sensor and/or an accelerometer.

4. System (100) according to any one of the preceding claims, wherein said display device (20) comprises wireless communication means (24) configured to communicate with an external entity (100', 300) in order to recover at least one item of external information (I3) relating to the external environment of said user (U).

5. System (100) according to any one of the preceding claims, wherein said display device (20) comprises acquisition means (25) configured to acquire at least one item of status information (I4) of the system (100).

6. System (100) according to any one of the preceding claims, wherein the control unit (22) is configured to control each of the light sources (21) in intensity and/or in colour.

7. System (100) according to any one of the preceding claims, wherein the plurality of light sources (21) comprises an LED or OLED strip.

8. System (100) according to any one of the preceding claims, the plurality of light sources (21) comprises an optical fibre.

9. Facial support (200) intended to be worn directly or indirectly on the head of the user (U) and comprising a system (100) according to any one of the preceding claims, wherein said visualisation window (11) is assembled integrally with said facial support (10).

10. Support (200) according to claim 9, which is in the form of a pair of goggles, a mask or a helmet.

11. Support (200) according to claim 9 or 10, which is provided with at least one strap (14) for holding in position said support (10) on the head of said user (U).
